# EUROPEAN PATENT APPLICATION

(11) **EP 4 516 768 A1**
(43) Date of publication of application: **05.03.2025**
(21) Application number: 23796558.7
(22) Date of filing: 28.04.2023
(51) Int. Cl.: C07C 21/18, C07C 21/185, C08J 3/00, C08J 11/12, C07C 17/367

(54) **METHOD FOR PRODUCING LOW-MOLECULAR-WEIGHT FLUORINE COMPOUND**

(30) Priority: 28.04.2022 JP 2022075504
(71) Applicant: Microwave Chemical Co., Ltd., Osaka-shi, Osaka 559-0025 (JP); DAIKIN INDUSTRIES, LTD., Osaka-shi, Osaka 530-0001 (JP); AGC INC., Chiyoda-ku, Tokyo 1008405 (JP)
(72) Inventor: TSUKAHARA, Yasunori, Osaka-shi, Osaka 559-0025 (JP); TAKAMOTO, Tamotsu, Osaka-shi, Osaka 559-0025 (JP); OGATA, Toshihiko, Osaka-shi, Osaka 559-0025 (JP); KANNO, Masahiro, Osaka-shi, Osaka 559-0025 (JP); DEGUCHI, Yukari, Osaka-shi, Osaka 559-0025 (JP); FUKUSHIMA, Kazuaki, Osaka-shi, Osaka 559-0025 (JP); KISHIKAWA, Yosuke, Osaka-Shi, Osaka 530-0001 (JP); HANDA, Shinya, Osaka-Shi, Osaka 530-0001 (JP); MATSUI, Motoshi, Osaka-Shi, Osaka 530-0001 (JP); SHIRAKAWA, Daisuke, Tokyo 100-8405 (JP); YAMAKI, Yasushi, Tokyo 100-8405 (JP); YAGISHITA, Masahito, Tokyo 100-8405 (JP)
(74) Representative: Müller-Boré & Partner Patentanwälte PartG mbB
(86) International application number: PCT/JP2023/016962
(87) International publication number: WO 2023/210826

(57) **Abstract**

The purpose of the present invention is to provide such a temperature difference that the temperature of a produced gas discharged from a reaction vessel becomes lower than the temperature in a decomposition reaction system, i.e., the temperature of a heated microwave absorber, in the decomposition of a fluororesin. Provided is a method for producing a low-molecular fluorine compound, the method comprising a decomposition step for heating a microwave absorber by the irradiation with microwaves in a reaction vessel provided with a carrier gas inlet and a decomposed gas outlet and then bringing the heated microwave absorber into contact with particles of a material containing a fluororesin to decompose the fluororesin into the low-molecular fluorine compound, in which the carrier gas temperature TI (°C) at the inlet is set to a temperature lower than the temperature TR (°C) of the heated microwave absorber. According to this method, it becomes possible to provide such a temperature difference that the temperature of a produced gas discharged from the reaction vessel becomes lower than the temperature in the decomposition reaction system, i.e., the temperature of the heated microwave absorber.

## Description

### Technical Field

The present invention relates to a technique for decomposing a fluororesin. More specifically, the present invention relates to a method for producing a low-molecular fluorine compound by thermal decomposition of a fluororesin using microwave irradiation heating.

### Background Art

Fluororesins are widely used in covering materials for heat-resistant electric wires, semiconductor manufacturing materials, electronic components, and the like utilizing their superior properties such as lubricity, heat resistance, and chemical resistance. In recent years, it has been actively studied to decompose and reuse synthetic resins including fluororesins. For example, Patent Document 1 proposes a chemical recycling method for extracting, from a material including PTFE, a tetrafluoroethylene low-molecular-weight monomer and a dimer thereof generated by thermally decomposing PTFE, the chemical recycling method including: a process of heating a fluidized bed reactor in which a fluidized bed formed of an inert particulate material is provided and in which the aforementioned material has been fed; and a process of introducing an inert gas as a fluidized gas into the fluidized bed reactor and extracting, together with the inert gas from the fluidized bed reactor, the tetrafluoroethylene low-molecular-weight monomer and the dimer thereof generated via heat decomposition of PTFE by that heating.

### Citation List

### Patent Literature

PTL 1: JP 2004-346000 A

### Summary of Invention

### Technical Problem

Regarding chemical recycling of a fluororesin, a technique as disclosed in Patent Literature 1 has been proposed, but control of the temperature environment to which a low-molecular fluorine compound such as an organic monomer generated from a fluororesin is exposed has not been studied, and there is still room for improvement in a method for decomposing a fluororesin.

The present inventors have focused on the fact that there is usually no difference between the temperature of the produced gas discharged from the reaction vessel and the temperature in the decomposition reaction system in the decomposition of the fluororesin, and have newly conceived, as a method of controlling the temperature environment, that the temperature of the produced gas discharged from the reaction vessel is intentionally controlled to be lower than the temperature in the decomposition reaction system.

Then, the present invention has employed microwave heating as a heating means in decomposition of a fluororesin and has an object to provide such a temperature difference (hereinafter also referred to as "temperature difference Δ2 (TR - TE)") that a temperature of a produced gas discharged from a reaction vessel (this temperature is hereinafter also referred to as "temperature TE") is lower than a temperature in a decomposition reaction system, namely, a temperature of a heated microwave absorber (this temperature is hereinafter also referred to as "temperature TR").

### Solution to Problem

As a result of intensive studies, the present inventors have found that the temperature difference recited above can be generated by setting the temperature at the carrier gas inlet into the reaction vessel to be lower than the ambient temperature of the reaction vessel. The present invention has been accomplished by repetitively conducting further studies on the basis of this finding.

In summary, the present invention provides aspects of invention as itemized below.

Item 1. A method for producing a low-molecular fluorine compound, comprising a decomposition step of, in a reaction vessel provided with a carrier gas inlet and a decomposed gas outlet, heating a microwave absorber by irradiation with microwaves and bringing the microwave absorber heated into contact with a material including a fluororesin to decompose the fluororesin into a low-molecular fluorine compound, wherein
a carrier gas temperature TI (°C) at the inlet is set to be lower than a temperature TR (°C) of the microwave absorber heated.

Item 2. The production method according to item 1, wherein a value obtained by dividing a temperature difference Δ1 (TR - TI) between the temperature TI and the temperature TR by the temperature TR is 0.5 or more.

Item 3. The production method according to item 1 or 2, wherein the temperature difference Δ1 (TR - TI) is more than 250°C.

Item 4. The production method according to any one of items 1 to 3, wherein the microwave absorber has a particle size d of 0.1 to 25 mm.

Item 5. The production method according to any one of items 1 to 4, wherein an introduction rate R of a carrier gas from the carrier gas inlet is 0.005 to 20 m/s.

Item 6. The production method according to any one of items 1 to 5, wherein
the microwave absorber is prepared in a form of a packed layer including particles of a material including the microwave absorber and the fluororesin, the packed bed being provided between the carrier gas inlet and the decomposed gas outlet, and
where a height H measured from a surface of the packed layer on a side facing the inlet to the outlet is 1, a ratio of a height h of the packed layer is 0.01 to 0.8.

Item 7. The production method according to any one of items 1 to 6, wherein a temperature TE (°C) of the produced gas discharged from the reaction vessel measured at the outlet is 20°C or more and less than 250°C.

### Advantageous Effects of Invention

According to the present invention, it becomes possible to provide a temperature difference such that the temperature of a produced gas discharged from a reaction vessel is lower than the temperature in a decomposition reaction system.

### Brief Description of Drawings

[Fig. 1] Fig. 1 shows a schematic view of the apparatus (parallel up-flow type) used in the production method of the present invention used in Examples.
[Fig. 2] Fig. 2 shows a schematic view of the apparatus (parallel down-flow type) used in the production method of the present invention used in Examples. Description of Embodiments

The method for producing a low-molecular fluorine compound of the present invention comprises a decomposition step of, in a reaction vessel provided with a carrier gas inlet and a decomposed gas outlet, heating a microwave absorber by irradiation with microwaves and bringing the microwave absorber heated into contact with particles of a fluororesin to decompose the fluororesin into a low-molecular fluorine compound, and is characterized in that a carrier gas temperature TI (°C) at the inlet is set to be lower than a temperature TR (°C) of the microwave absorber heated. Hereinafter, the method for producing a low-molecular fluorine compound of the present invention will be described in detail.

### [1. Fluororesin]

In the present invention, a fluororesin is used as a raw material of a low-molecular fluorine compound. The fluororesin is any fluorine compound having a molecular weight of 500 or more and a fluorine content (weight basis) of 57% or more.

Examples of the fluororesin include fluorinated polyolefins and fluorinated polyethers.

Fluorinated polyolefins are polymers or oligomers having repeating units derived from fluorinated olefins.

Examples of the fluorinated olefins include tetrafluoroethylene (TFE), hexafluoropropylene (HFP), vinylidene fluoride (VDF), chlorotrifluoroethylene (CTFE), partially fluorinated or perfluorovinyl ether, and partially fluorinated or perfluoroallyl ether.

Examples of the perfluorovinyl ether include compounds represented by the general formula CF₂=CF-O-R_{f}, wherein R_{f} represents a perfluorinated linear, cyclic, or branched aliphatic group optionally containing one or more oxygen atoms. When R_{f} contains one or more oxygen atoms, examples of the R_{f} include a group represented by - (R^{a}ᵣO)ₙ(R^{b}ᵣO)ₘR^{c}_{f}, wherein R^{a}_{f} and R^{b}_{f} are different linear or branched chain or cyclic perfluoroalkylene groups having 1 to 6 carbon atoms, in particular 2 to 6 carbon atoms, m and n are independently 0 to 10, and R^{c}_{f} is a perfluoroalkyl group having 1 to 6 carbon atoms. When R_{f} contains no oxygen atoms, examples of the perfluorovinyl ether include perfluoromethyl vinyl ether (PMVE), perfluoropropyl vinyl ether, and perfluoroisopropyl vinyl ether.

Examples of the perfluorovinyl ether include perfluoro(methyl vinyl) ether (PMVE), perfluoro(ethyl vinyl) ether (PEVE), perfluoro(n-propyl vinyl) ether (PPVE-1), perfluoro-2-propoxypropyl vinyl ether (PPVE-2), perfluoro-3-methoxy-n-propyl vinyl ether, perfluoro-2-methoxy-ethyl vinyl ether, CF₃-(CF₂)₂-O-CF(CF₃)-CF₂-O-CF(CF₃)-CF₂-O-CF=CF₂, CF₂=CF-O-(CF₂)₃OCF₃, and CF₂=CFO(CF₂)₂OCF₃.

Examples of the perfluoroallyl ether include compounds represented by the general formula CF₂=CF-CF₂-O-R_{f}, wherein R_{f} is the same as R_{f} described above for the perfluorovinyl ether.

The fluorinated polyolefin may be a polymer or oligomer composed of repeating units derived from a fluorinated olefin, or alternatively may be a polymer or oligomer further containing repeating units derived from a non-fluorinated olefin in addition to repeating units derived from a fluorinated olefin. The non-fluorinated olefin is not particularly limited, and examples thereof include C₂ to C₈ olefins, and more preferably include ethylene (E) and propylene (P). Among these fluorinated polyolefins, polymers or oligomers composed of repeating units derived from a fluorinated olefin (namely, fluorinated polyolefins that have a molar ratio of repeating units derived from a non-fluorinated olefin is 0%) are preferred.

Suitable examples of the fluorinated polyolefin include homopolymers of TFE and the following copolymers, namely, polymers containing repeating units derived from TFE and HFP; TFE, HFP and VDF; TFE and CTFE; TFE, CTFE and E or P; TFE and PMVE, PEVE, PPVE-1 or PPVE-2; TFE, E and HFP; CTFE and E or P.

Fluorinated polyethers are polymers or oligomers having fluorinated oxyalkylene repeating units.

The fluorinated oxyalkylene repeating unit is represented by -CₗX₂ₗO-, wherein l is an integer of 1 or more, X is hydrogen or fluorine, the unit is linear or branched, and l and X may be different in each repetitive occurrence.

The fluororesin may be a fluorinated non-ionomer or alternatively may be a fluorinated ionomer (a fluoropolymer having a plurality of -SO₃- and/or -COO- groups as pendant or terminal groups). As the fluorinated ionomer, one used for the preparation of a membrane in a fuel cell or in an electrochemical reaction can be used without particular limitation.

The fluororesin may have a hydrogen atom or a functional group containing an atom other than fluorine at a terminal. The functional group containing an atom other than fluorine is not particularly limited, and examples thereof include an iodine group, a bromine group, a chlorine group, a hydroxy group and an ester thereof, and a vinyl group.

The fluororesins may be used singly or two or more types thereof may be used in combination.

### [2. Material containing fluororesin]

The fluororesin is subjected to a decomposition step in the form of a material containing it. The material may be made of only a fluororesin or may contain components other than the fluororesin. Examples of the other components include resin additives to be blended in fluororesin compositions, and more specifically include a filler, a dye, a lubricant, a curing agent, a curing accelerator, a thermally conductive agent, and an electrically conductive agent.

The properties of the material containing the fluororesin in the decomposition step are determined according to the conditions in the decomposition step, and are not particularly limited. When the material containing the fluororesin is a solid, the material is used in the form of particles. The particles are obtained by processing a fluororesin material into the form of particles by an arbitrary means. Examples of the means include a milling device and a pulverizing device.

The size of the particles is not particularly limited, and is, for example, 20 µm to 100 mm, preferably 100 µm to 10 mm, and more preferably 250 µm to 5 mm.

The material containing the fluororesin may be one obtained via pretreatment that can be performed prior to the decomposition step. Such pretreatment is not particularly limited as long as a material containing a fluororesin can be obtained, and examples thereof include treatment of separating and removing a member not containing a fluororesin and/or a material other than a fluororesin from a material to be recycled, treatment of removing oil and/or dust, and fluorination treatment of a polymer material (treatment of a polymer material with F₂ gas to convert a C-H bond into a C-F bond).

A method for feeding the material containing the fluororesin into the reaction vessel is not particularly limited, and may be appropriately selected by those skilled in the art. For example, when the material containing the fluororesin is particles, it is preferable to feed the particles into the reaction vessel by, for example, gravity feeding, and when the material containing the fluororesin is a liquid, the liquid can be fed into the reaction vessel by, for example, spraying.

### [3. Reaction Vessel]

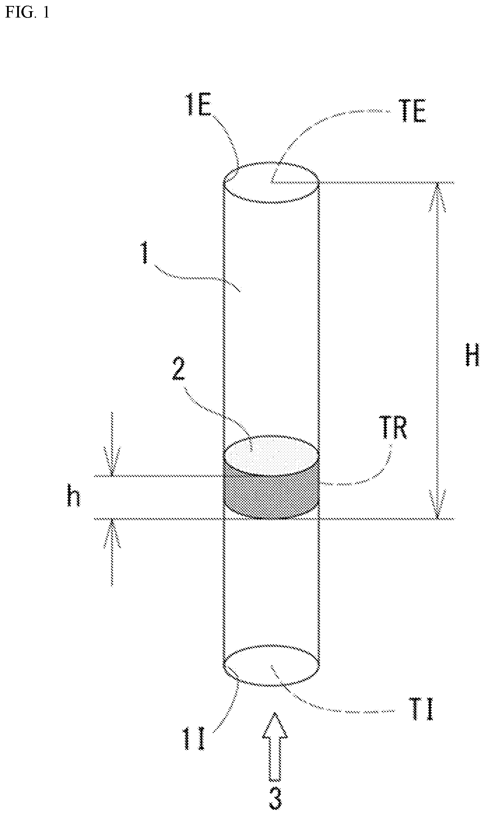

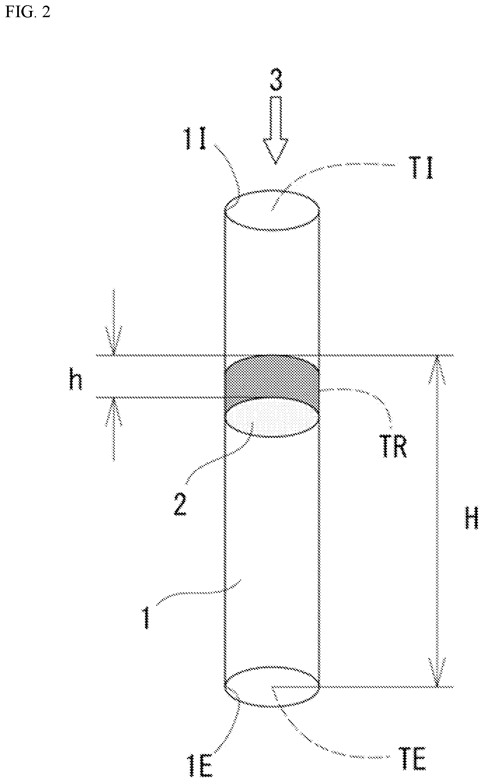

As the reaction vessel, a vessel provided with a carrier gas inlet and a decomposed gas outlet for introducing and discharging a gas flow is used. In the present invention, the flow type of the gas flow is preferably a parallel flow type, and specifically, a parallel up-flow type (see Fig. 1) or a parallel down-flow type (see Fig. 2).

More specifically, the reaction vessel in the present invention can be incorporated into an apparatus according to a common catalyst reactor, and in the apparatus, a microwave absorber is used instead of the catalyst in the common catalyst reactor.

Furthermore, since microwaves are used as the heating means in the present invention, the apparatus may include a microwave generator. As the microwave generator, a publicly known device may be appropriately selected, and examples of such a device include a diode, a magnetron, a gyrotron, a traveling wave tube, a klystron, a ubitron, and an amplitron.

### [4. Microwave absorber]

### [4-1. Examples of microwave absorber]

The microwave absorber is not particularly limited as long as the microwave absorber absorbs the irradiated microwave to generate heat at a temperature at which the fluororesin can be decomposed, and is in a solid state under the decomposition reaction conditions of the fluororesin and has chemically inert characteristics. The temperature TR of the microwave absorber heated in the decomposition step constitutes the temperature condition for advancing the decomposition reaction of the fluororesin and corresponds to the temperature in the reaction system of the decomposition reaction of the fluororesin.

Examples of the microwave absorber include metals, metal salts, intermetallic compounds, carbon, and carbides which all have the characteristics described above. Preferably, a substance having a density of, for example, 1 to 10 g/cm³, preferably 1.5 to 7 g/cm³ can be used as the microwave absorber. In addition, preferably, a substance having microwave absorbing ability with a dielectric loss of, for example, 0.01 to 30, preferably 0.1 to 11 can be used as the microwave absorber.

Among more specific examples of the microwave absorber, examples of the metal include single metal such as Ni, Pt, Co, or Pt, and an alloy such as Pt/Cu or Pt/Re. Examples of the metal salt include metal oxides, metal hydroxides, metal halides, and silicides. Examples of the intermetallic compound include molybdenum silicide and titanium boride. Examples of the carbon include graphite such as expanded graphite, activated carbon, carbon fiber, carbon nanotube, and carbon black. Examples of the carbide include silicon carbide, boron carbide, titanium carbide, and zirconium carbide. These microwave absorbers may be used singly or two or more types thereof may be used in combination. Among these microwave absorbers, carbides are preferred, and silicon carbide is more preferred.

### [4-2. Particle size d of microwave absorber]

In the present invention, the microwave absorber is preferably used in the form of particles. The particle size (hereinafter also referred to as "particle size d") of the microwave absorber particles is not particularly limited, and is, for example, 0.1 to 25 mm. In addition, the particle size d in a case where the mode of the packed layer described later is a fixed bed is preferably 0.1 to 25 mm, more preferably 1 to 25 mm, still more preferably 2.8 to 25 mm, and further preferably 4.8 to 25 mm from the viewpoint of further increasing the temperature difference Δ2 (TR - TE), and the upper limit of the range of the particle sizes d may be 10 mm or less or 8 mm or less. When the mode of the packed layer described later is a fluidized bed, the particle size d is preferably 0.5 to 25 mm, more preferably 1 to 25 mm, still more preferably 2 to 10 mm, further preferably 2.8 to 25 mm, and still further preferably 4.8 to 25 mm from the viewpoint of further increasing the temperature difference Δ2 (TR - TE), and the upper limit of the range of the particle size d may be 15 mm or less or 10 mm or less. In the present invention, the particle size d is an average of measurement values obtained by measuring, with a gauge, the maximum diameters in a fixed direction of randomly selected 20 particles. For the measurement with a gauge, visual observation, an optical microscope, or an electron microscope is used depending on the size of the particles.

### [4-3. Mode of packed layer]

A more specific mode of the microwave absorber during the decomposition reaction may be either a fixed bed or a fluidized bed, but from the viewpoint of more easily controlling the temperature difference Δ2 (TR - TE), a fixed bed is preferable.

The microwave absorber can be prepared in the reaction vessel in the form of a packed layer of a mixture including the microwave absorber and particles of a material including the fluororesin before the decomposition step.

The use amount of the microwave absorber with respect to the amount of the particles of the material including the fluororesin is not particularly limited, and is, for example, 0.1 to 1000 parts by weight, and preferably 1 to 10 parts by weight with respect to 1 part by weight of the particles of the material including the fluororesin.

The height of the packed layer of the mixture including particles of the material including the microwave absorber and the fluororesin at the initial stage (before starting the decomposition step) (this height is hereinafter also described as "height h") is also not particularly limited. However, where the height from the surface of the packed layer located on the inlet side (the lower surface of the packed layer is taken when the flow type of the carrier gas is the parallel up-flow type, whereas the upper surface of the packed layer is taken when the flow type of the carrier gas is the parallel down-flow type) to the outlet of the vessel (this height is hereinafter also described as "height H") is 1 is, the ratio (h/H) of the height h of the packed layer is, for example, 0.01 to 0.8, and from the viewpoint of further increasing the temperature difference Δ2 (TR - TE), the ratio is preferably 0.01 to 0.5, more preferably 0.01 to 0.4, and still more preferably 0.01 to 0.3.

Specific examples of the height h of the packed layer include, for example, 0.1 to 200 mm, and from the viewpoint of further increasing the temperature difference Δ2 (TR - TE), the height h is preferably 1 to 100 mm, more preferably 3 to 50 mm, and still more preferably 5 to 30 mm. In addition, the height h when the mode of the packed layer is a fixed bed is preferably 3 to 50 mm, more preferably 5 to 30 mm, and the height h when the mode of the packed layer is a fluidized bed is preferably 5 to 30 mm.

### [5. Carrier gas]

### [5-1. Examples of carrier gas]

The carrier gas introduced from the carrier gas inlet of the reaction vessel is not particularly limited as long as it has a chemically inert characteristic under the decomposition reaction conditions of the fluororesin, and examples thereof include water vapor, nitrogen, and a rare gas (Xe, Ar, Ne, He, etc.). These carrier gases may be used singly or two or more types thereof may be used in combination. Among these carrier gases, water vapor and nitrogen are preferable.

### [5-2. Temperature TI of carrier gas at inlet]

In the present invention, to create the temperature difference Δ2 (TR - TE), the carrier gas temperature at the inlet (hereinafter also referred to as "temperature TI", the unit is "°C") is set to be lower than the temperature of the microwave absorber heated (hereinafter also referred to as "temperature TR"; the unit is "°C").

To create the temperature difference Δ2 (TR - TE), the temperature TI is preferably set to ensure a sufficient difference from the temperature TR. Specifically, the temperature TI may be set such that a value obtained by dividing a difference from the temperature TR (this temperature difference is hereinafter also described as "temperature difference Δ1 (TR - TI)") by the temperature TR is, for example, 0.5 or more, preferably 0.85 or more, more preferably 0.9 or more, still more preferably 0.93 or more, and further preferably 0.94 or more. The upper limit of the value obtained by dividing the temperature difference Δ1 (TR - TI) by the temperature TR is not particularly limited, and may be, for example, 0.96 or less.

The temperature TI may be set such that the temperature difference Δ1 (TR - TI) is, for example, more than 250°C, preferably 300°C or more, and more preferably 350°C or more. The upper limit of the temperature difference Δ1 (TR - TI) is not particularly limited, and is, for example, 490°C or lower.

In the present invention, the carrier gas at the inlet may be preliminarily heated or may not be preliminarily heated. That is, in the present invention, the production method may or may not include the step of preliminarily heating the carrier gas. Examples of the temperature TI include, for example, 10 to 400°C, preferably 15 to 300°C, and more preferably 20 to 200°C. More specifically, preferable examples of the temperature TI in the case of not preliminarily heating include 10 to 30°C or 15 to 25°C, and preferable examples in the case of preliminarily heating include 80 to 400°C, preferably 90 to 200°C, and more preferably 100 to 150°C.

### [5-3. Introduction rate R of carrier gas]

The introduction rate (hereinafter also referred to as "introduction rate R") of the carrier gas from the carrier gas inlet is not particularly limited, and is, for example, 0.005 to 20 m/s, and from the viewpoint of further increasing the temperature difference Δ2 (TR - TE), the introduction rate is preferably 0.1 to 20 m/s, more preferably 0.2 to 18 m/s, still more preferably 0.3 to 15 m/s, still more preferably 0.4 to 15 m/s.

The introduction rate R may also be set in consideration of the particle size d of the microwave absorber, and the like according to whether the mode of the packed layer is a fixed bed or a fluidized bed. For example, the introduction rate R set when the mode of the packed layer is a fixed bed is preferably 0.005 to 2 m/s, and from the viewpoint of further increasing the temperature difference Δ2 (TR - TE), the introduction rate R is more preferably 0.2 to 1.8 m/s, still more preferably 0.2 to 1.6 m/s, and further preferably 0.2 to 0.8 m/s. The introduction rate R in the case where the mode of the packed layer is a fluidized bed is preferably 0.005 to 20 m/s, and from the viewpoint of further increasing the temperature difference Δ2 (TR - TE), the introduction rate R is more preferably 0.01 to 18 m/s, still more preferably 0.4 to 15 m/s, and further preferably 1 to 14 m/s.

### [6. Decomposition reaction]

In the decomposition reaction, the microwave absorber is heated by irradiation with microwaves in the reaction vessel, and the microwave absorber heated is brought into contact with particles of a material containing a fluororesin to decompose the fluororesin into a low-molecular fluorine compound.

The temperature TR of the microwave absorber heated is just required to be a temperature at which the fluororesin is decomposed. The temperature TR is specifically, for example, 280 to 1500°C, preferably 380 to 1000°C, and more preferably 480 to 850°C. The microwave irradiation conditions (wavelength and frequency) for heating the microwave absorber may be appropriately set according to the temperature TR.

The temperature TR is a temperature obtained by measuring a temperature at a central position in the height h direction of the packed layer from the side surface side of the reaction vessel in a state where the reaction vessel is thermally insulated. The heat insulation of the reaction vessel at the time of measuring the temperature TR is performed by surrounding the reaction vessel with ceramic wool so as to have a thickness of 20% of the maximum outer peripheral width of the reaction vessel. For the temperature measurement, an optical fiber thermometer or a non-contact thermometer can be used. This heat insulation method constitutes the measurement conditions of the temperature TR specified in the present invention, and the production method of the present invention is not limited to the mode in which this heat insulation method is performed.

The low-molecular fluorine compound, which is a product of the decomposition reaction, is a decomposition product of the fluororesin as a raw material, and the molecular weight thereof is not particularly limited as long as it is lower in molecular weight than the fluororesin as a raw material. In the present invention, the low-molecular fluorine compound, which is a decomposition product of the fluororesin, is a gas (produced gas) at the temperature TE. A specific temperature of the temperature TE is not particularly limited, but the temperature TE is preferably 20°C or more and less than 250°C, more preferably 50 to 230°C, and still more preferably 95 to 200°C since in the present invention the temperature TE can be obtained at a relatively low temperature by providing the temperature difference Δ2 (TR - TE).

The temperature TE in the present invention is a temperature which the decomposed gas (produced gas) having passed through the packed layer has when the decomposed gas reaches the decomposed gas outlet of the reaction vessel without being cooled by any rapid cooling method. The rapid cooling method as used herein refers to a method of cooling the decomposed gas so as to have a temperature lower than a temperature in the case of natural cooling (namely, a temperature which the decomposed gas having passed through the packed layer exhibits when the decomposed gas is naturally cooled before reaching the decomposed gas outlet of the reaction vessel, and the decomposed gas reaches the decomposed gas outlet). Specific methods are known in the art, and examples thereof include a method of expanding the decomposed gas (produced gas), a method of cooling the decomposed gas using a separately prepared cooling gas, and a method of cooling the decomposed gas using a cooling liquid.

Specifically, the temperature difference Δ2 (TR - TE) is preferably 50°C or more, more preferably 100°C or more, and still more preferably 250°C or more. The upper limit is not particularly limited, but is, for example, 400°C.

Examples of the low-molecular fluorine compound include compounds represented by the general formula CF₂=CF-R^{d}_{f} wherein R^{d}_{f} represents F or a perfluoroalkyl group having 1 to 10, preferably 1 to 5, carbon atoms, and preferably include tetrafluoroethylene (TFE) and hexafluoropropylene (HFP). Examples of the fluorinated olefins include vinylidene fluoride (VDF), chlorotrifluoroethylene (CTFE), perfluorovinyl ether, and perfluoroallyl ether. Still other examples of the low-molecular fluorine compound include octafluorocyclobutane.

The low-molecular fluorine compound (produced gas), which is a product, is discharged from the outlet of the reaction vessel by a carrier gas flow, and then is collected. As a collecting method, the produced gas may be appropriately cooled and/or concentrated.

During the decomposition step, additional feed of the material containing the fluororesin may not be performed (that is, the process may be a batch process), or the fluororesin may be additionally fed continuously or intermittently.

### Examples

Hereinafter, the present invention will be described more specifically with reference to Examples, but the present invention is not limited the following Examples.

### [Test Example 1]

### (Cases of Examples 1 to 10)

The parallel up-flow type apparatus illustrated in Fig. 1 was prepared, and the packed layer 2 made of a microwave absorber having a prescribed particle size d was provided between the carrier gas inlet 1I and the decomposed gas outlet 1E of the cylindrical reaction vessel 1. The height from the lower surface of the packed layer 2 to the outlet 1E was defined as H, and the height of the packed layer was defined as h.

### (Case of Example 11)

The parallel down-flow type apparatus illustrated in Fig. 2 was prepared, and the packed layer 2 containing particles of a material containing a microwave absorber having a prescribed particle size d and a fluororesin (PTFE) was provided between the carrier gas inlet 1I and the decomposed gas outlet 1E of the cylindrical reaction vessel 1. The height from the upper surface of the packed layer 2 to the outlet 1E was defined as H, and the height of the packed layer was defined as h.

### (Common to Examples 1 to 11)

While the carrier gas (nitrogen) 3 was introduced from the inlet 1I at a prescribed carrier gas temperature TI and a prescribed introduction rate R, the microwave absorber (silicon carbide) in the packed layer 2 was heated to a prescribed temperature TR corresponding to the decomposition reaction temperature of the fluororesin by a microwave generator (not shown), and the temperature TE of the generated gas at the outlet 1E was measured. The temperature TR was measured by surrounding the reaction vessel with ceramic wool so as to have a thickness of 20% of the outer diameter of the vessel to insulate the reaction vessel, and measuring the temperature at the central position in the height h direction of the packed layer from the side surface side of the reaction vessel using a non-contact thermometer. The set conditions and measurement results are shown in Tables 1 and 2.

**[Table 1]**

| | Example | | |
|---|---|---|---|
| | 1 | 2 | 3 |
| Carrier gas temperature TI (°C) | 20 | | |
| Microwave absorber temperature TR (°C) | 400 | 300 | 300 |
| Microwave output (kW) | 1.3 | 0.7 | 3 |
| Microwave absorber | SiC | | |
| Microwave absorber particle size d (mm) | 3 | | |
| Container inner diameter (mm) | 50 | | |
| Height H from packed layer lower surface to outlet (mm) | 470 | | |
| Height h of packed layer (mm) | 20 | | |
| h/H | 0.043 | | |
| Carrier gas introduction rate R (m/s) | 0.8 | 0.8 | 1.3 |
| Fluidized bed/ Fixed bed | Fixed | Fixed | Fluidized |
| Outlet temperature TE (°C) | 150 | 100 | 180 |
| Δ1 (TR - TI)/TR | 0.95 | 0.93 | 0.93 |
| Δ1 (TR - TI) (°C) | 380 | 280 | 280 |
| Δ2 (TR - TE) (°C) | 250 | 200 | 120 |

**[Table 2]**

| | Example | | | | | | | | | | |
|---|---|---|---|---|---|---|---|---|---|---|---|
| | 4 | 5 | 6 | 7 | 8 | 4 | 9 | 6 | 7 | 10 | 11 |
| Carrier gas temperature TI (°C) | 20 | | 20 | | 20 | | 20 | | 20 | | 21 |
| Microwave absorber temperature TR (°C) | 501 | 501 | 501 | 500 | 501 | 501 | 502 | 501 | 500 | 500 | 400 |
| Microwave absorber | SiC | | SiC | | SiC | | SiC | | SiC | | SiC |
| Microwave absorber particle size d (mm) | 5 | 10 | 5 | 10 | 5 | | 5 | | 10 | | 5 |
| Container inner diameter (mm) | 12 | | 12 | | 12 | | 12 | | 12 | | 12 |
| Height H from packed layer lower surface to outlet (mm) | 110 | | 110 | | 110 | | 110 | | 110 | | 110^{(*)} |
| Height h of packed layer (mm) | 20 | | 20 | | 10 | 20 | 10 | 20 | 20 | 30 | 30 |
| h/H | 0.19 | | 0.19 | | 0.09 | 0.19 | 0.09 | 0.19 | 0.19 | 0.27 | 0.27 |
| Carrier gas introduction rate R (m/s) | 0.44 | | 1.5 | | 0.44 | | 1.5 | | 1.5 | | 0.015 |
| Fluidized bed/ Fixed bed | Fixed | | Fixed | | Fixed | | Fixed | | Fixed | | Fixed |
| Outlet temperature TE (°C) | 232 : | 210 | 189 | 174 | 179 | : 232 | 149 | 189 | 174 : | 210 | 23 |
| Δ1 (TR - TI)/TR | 0.96 | 0.96 | 0.96 | 0.96 | 0.96 | 0.96 | 0.96 | 0.96 | 0.96 | 0.96 | 0.95 |
| Δ1 (TR - TI) (°C) | 481 \| | 481 | 481 | 480 | 481 | 481 | 482 | 481 | 480 \| | 480 | 379 |
| Δ2 (TR - TE) (°C) | 270 | 290 | 312 | 326 | 322 | 270 | 353 | 312 | 326 | 290 | 377 |

| | | | | | | | | | | | |
|---|---|---|---|---|---|---|---|---|---|---|---|
| (*) Only in Example 12, the height H is the height from the packed layer upper surface to the outlet. | | | | | | | | | | | |

As shown in Examples 1 to 11, it has been confirmed that there effectively occurred such a temperature difference Δ2 (TR - TE) that the temperature TE of the produced gas discharged from the reaction vessel was lower than the temperature in the fluororesin decomposition reaction system, namely, the temperature TR of the microwave absorber heated. Therefore, according to Examples 1 to 11, it has been confirmed that the temperature can be easily controlled in such a manner that the temperature of the produced gas discharged from the vessel is low. Such temperature control improves the handleability of the produced gas. According to Example 11, tetrafluoroethylene, hexafluoropropylene, and octafluorocyclobutane were obtained in the form of a mixed produced gas, and the sum total of 100 parts by weight of these three produced gases was accounted for by 53 parts by weight of tetrafluoroethylene, 18 parts by weight of hexafluoropropylene, and 29 parts by weight of octafluorocyclobutane.

### [Test Example 2]

In the parallel up-flow type apparatus of Fig. 1, a packed layer 2 made of a microwave absorber having a prescribed particle size d was provided between a carrier gas inlet 1I and a decomposed gas outlet 1E of a cylindrical reaction vessel 1, and while a carrier gas (nitrogen) 3 was introduced from the inlet 1I at a prescribed temperature TI and a prescribed introduction rate R, the microwave absorber in the packed layer 2 was heated to a prescribed temperature TR by a microwave generator (not shown), and a temperature TE at the outlet 1E of the decomposed gas when a decomposition reaction of the fluororesin was performed was analyzed and derived. Note that a fluidized bed B refers to a uniform fluidized bed (i.e., such a fluidized bed that during a decomposition reaction, the particles constituting the packed layer fly up, as a result, the height of the packed layer becomes greater than the initial height h, but no air bubbles have been formed), and a fluidized bed C refers to a bubble fluidized bed (i.e., such a fluidized bed that during a decomposition reaction, the particles constituting the packed layer fly up, as a result, the height of the packed layer becomes greater than the initial height h, and air bubbles have been formed). The set conditions and analysis results are shown in Table 3.

**[Table 3]**

| | Example | | | | | |
|---|---|---|---|---|---|---|
| | 12 | 13 | 14 | 15 | 16 | 17 |
| Carrier gas temperature TI (°C) | 20 | 20 | 20 | 20 | 20 | 20 |
| Microwave absorber temperature TR (°C) | 350 | 350 | 350 | 350 | 350 | 350 |
| Microwave absorber | SiC | | C | | BaTiO₃ | |
| Microwave absorber particle size d (mm) | 2 | 3 | 3 | 3 | 3 | 3 |
| Container inner diameter (mm) | 300 | 300 | 300 | 300 | 300 | 300 |
| Height H from packed layer lower surface to outlet (mm) | 2000 | 2000 | 2000 | 2000 | 2000 | 2000 |
| Height h of packed layer (mm) | 20 | 20 | 20 | 20 | 20 | 20 |
| h/H | 0.01 | 0.01 | 0.01 | 0.01 | 0.01 | 0.01 |
| Carrier gas introduction rate R (m/s) | 8.5 | 10.5 | 13.3 | 7.8 | 10.5 | 15 |
| Fluidized bed/ Fixed bed | Fluidized B | Fluidized B | Fluidized C | Fluidized C | Fluidized B | Fluidized B |
| Outlet temperature TE (°C) | 200 | 100 | 50 | 100 | 150 | 150 |
| Δ1 (TR - TI)/TR | 0.94 | 0.94 | 0.94 | 0.94 | 0.94 | 0.94 |
| Δ1 (TR - TI) (°C) | 330 | 330 | 330 | 330 | 330 | 330 |
| Δ2 (TR - TE) (°C) | 150 | 250 | 300 | 250 | 200 | 200 |

As shown in Examples 12 to 17, it has been reasonably confirmed that there effectively occurs such a temperature difference Δ2 (TR - TE) that the temperature TE of the produced gas discharged from the reaction vessel is lower than the temperature in the fluororesin decomposition reaction system, namely, the temperature TR of the microwave absorber heated. Therefore, according to Examples 12 to 17, it has been reasonably confirmed that the temperature can be easily controlled in such a manner that the temperature of the produced gas discharged from the vessel is low.

### Reference Signs List

1 Reaction vessel
1I Carrier gas inlet
1E Carrier gas outlet
TI Carrier gas temperature at inlet 1I
TE Produced gas temperature at outlet 1E
2 Packed layer
TR Temperature of microwave absorber heated
3 Carrier gas
H Height of packed layer 2 from surface on inlet 1I side to outlet 1E
h Height of packed layer

## Claims

1. A method for producing a low-molecular fluorine compound, comprising a decomposition step of, in a reaction vessel provided with a carrier gas inlet and a decomposed gas outlet, heating a microwave absorber by irradiation with microwaves and bringing the microwave absorber heated into contact with a material including a fluororesin to decompose the fluororesin into a low-molecular fluorine compound, wherein
a carrier gas temperature TI (°C) at the inlet is set to be lower than a temperature TR (°C) of the microwave absorber heated.

2. The production method according to claim 1, wherein a value obtained by dividing a temperature difference Δ1 (TR - TI) between the temperature TI and the temperature TR by the temperature TR is 0.5 or more.

3. The production method according to claim 1, wherein the temperature difference Δ1 (TR - TI) is more than 250°C.

4. The production method according to claim 1, wherein the microwave absorber has a particle size d of 0.1 to 25 mm.

5. The production method according to claim 1, wherein an introduction rate R of a carrier gas from the carrier gas inlet is 0.005 to 20 m/s.

6. The production method according to claim 1, wherein
the microwave absorber is prepared in a form of a packed layer including particles of a material including the microwave absorber and the fluororesin, the packed layer being provided between the carrier gas inlet and the decomposed gas outlet, and
where a height H measured from a surface of the packed layer on a side facing the inlet to the outlet is 1, a ratio of a height h of the packed layer is 0.01 to 0.8.

7. The production method according to claim 1, wherein a temperature TE (°C) of the produced gas discharged from the reaction vessel measured at the outlet is 20°C or more and less than 250°C.
